# EUROPEAN PATENT APPLICATION

(11) **EP 2 974 783 A1**
(43) Date of publication of application: **20.01.2016**
(21) Application number: 15176811.6
(22) Date of filing: 15.07.2015
(51) Int. Cl.: B01D 53/32, A61L 9/22, B03C 3/08

(54) **PLASMA ELECTRODE DEVICE AND AIR CONDITIONING APPARATUS**

(30) Priority: 16.07.2014 KR 20140089498
(71) Applicant: LG Electronics Inc., Yeongdeungpo-gu Seoul 150-721 (KR)
(72) Inventor: JANG, Jaesoo, 153-802 Seoul (KR); JUNG, Yeekyeong, 153-802 Seoul (KR); SUNG, Bongjo, 153-802 Seoul (KR)
(74) Representative: Vossius & Partner Patentanwälte Rechtsanwälte mbB

(57) **Abstract**

A plasma device and an air conditioner including a plasma device are provided. The plasma device may include a substrate body (100), a first electrode (110) disposed on one or a first surface of the substrate body to perform plasma discharge, and a second electrode disposed on the other or a second surface of the substrate body to act with the first electrode. The substrate body may include a third electrode that acts with the first electrode or the second electrode to perform the plasma discharge, and an insulator that surrounds the third electrode.

## Description

In recent years, introduction of external gas into buildings may be minimized to reduce energy consumption. Accordingly, due to air-tight buildings, indoor air pollution in the buildings is becoming more serious. As a result, various kinds of judiciary regulations with respect to indoor pollutants are being increasingly enforced.

While home appliances installed in homes or companies operate, indoor pollutants may be generated and deposited within the home appliances or discharged from the home appliances. The indoor pollutants may cause an unpleasant smell and have a bad impact on a user's health.

For example, in a case of home appliances using air containing moisture or water, such as air conditioners, dehumidifiers, air cleaners, refrigerators, or washing machines, pollution due to dust or microorganisms inside or outside the home appliances may occur. In detail, the indoor pollutants may be classified into (1) particle pollutants, such as fine dust and asbestos, for example, (2) gas pollutants, such as voltaic organic compounds (VOCs), for example, and (3) biological pollutants, such as viruses, and molds, for example.

To remove the indoor pollutants, surface discharge induced plasma chemical processing may be used. In general, the surface discharge induced plasma chemical processing may be understood as or refer to a process in which a strong plasma region is formed on a surface of a device through high frequency discharging using ceramic to generate a large amount of OH radicals and ozone, thereby removing the pollutants using the generated radicals and ozone.

The present Applicant has filed an application (hereinafter, referred to as a "related art") as follows with respect to the above-described technology, Korean Patent Registration No. 10-0657476, entitled "Surface Discharge Induced Air Purifier" and registered on December 7, 2006, which is hereby incorporated by reference. The air purifier according to the related art includes a plasma device including a discharge electrode disposed on a top surface of two sheets of insulating dielectrics, which are attached to each other, a ground electrode disposed between the two sheets of insulating dielectrics, and a coating layer that shields the discharge electrode to prevent the discharge electrode from being directly exposed to air.

Each of the insulating dielectrics may be coated with an insulating material. For example, the insulating material may include ceramic. When the insulating material is applied, or the coating layer is formed, it may be necessary to generate uniform discharge so that a surface of the electrode is uniformly coated.

However, in the case of the plasma device according to the related art, the discharge may be performed using the two sheets of electrodes. Thus, it may be difficult to realize a uniform coating thickness, and unevenness which is above an allowable range may occur on the surface during the coating process.

Also, to maintain a uniform distance between the electrodes, it may be necessary to provide a separate fixing unit or device. In addition, it may be difficult to apply the plasma device in a curved structure. Also, when a passage is formed for actual application, as air flows in a vertical direction, a pressure loss may be high.

It is an object to provide a plasma electrode device having better characteristics and an air conditioning apparatus comprising a plasma electrode device.

This object is achieved with the features of the independent claims. The dependent claims relate to further aspects of the invention.

Embodiments provide a plasma electrode device that is capable of removing pollutants.

Embodiments disclosed herein provide a plasma device that is capable of removing pollutants.

Embodiments disclosed herein provide a plasma device that may include a substrate body; a first discharge electrode disposed on one or a first surface of the substrate body to perform plasma discharge; and a second discharge electrode disposed on the other or a second surface of the substrate body to act with the first discharge electrode. The substrate body may include a ground electrode that acts with the first or second discharge electrode to perform the plasma discharge, and an insulator that surrounds the ground electrode.

The insulator may include a base on which the ground electrode may be seated; a side surface part or side surface that extends from each of both sides of the base to surround a side surface of the ground electrode; and a top surface part or top surface that extends from the side surface part to surround a top surface of the ground electrode. The insulator may be formed of an epoxy resin.

A photocatalyst part or photocatalyst which is activated by visual light to decompose pollutants or reduce an amount of ozone may be disposed on at least one surface of the insulator. The photocatalyst part may include silver phosphate (Ag₃PO₄), titanium dioxide (TiO₂), and an inorganic binder.

The second discharge electrode may include a discharge electrode part or pad to which power may be applied; at least one pattern frame disposed on a bottom surface of the substrate body; and at least one discharge tip disposed on the pattern frame. The first discharge electrode may include a frame that defines an edge of one surface of the substrate body, and a plurality of branches branched from the frame to form a pattern.

A first electrode part or pad to which power may be applied may be disposed on one surface of the substrate body, and the first discharge electrode may be connected to a connection line that extends from the first electrode part. A second electrode part or pad to which power may be applied may be disposed on the other surface of the substrate body, and the second discharge electrode may be connected to a connection line that extends from the second electrode part.

Air may flow along one surface of the substrate body on which the first discharge electrode is disposed to generate ions through reaction.

Embodiments disclosed herein further provide an air conditioning apparatus or air conditioner that may include a main body having a suction hole through which air may be suctioned and a discharge hole through which the air suctioned in through the suction hole may be discharged; a fan disposed in the main body to blow the air; a charging device or charger coupled to the main body outside of the main body to charge dust in the air; and a plasma device disposed between the suction hole and the discharge hole within the main body to generate a large amount of ions. The plasma device may include a first discharge electrode disposed on one or a first surface of the substrate body to perform plasma discharge; and a second discharge electrode disposed on the other or a second surface of the substrate body to act with the first discharge electrode. The substrate body may include a ground electrode that acts with the first or second discharge electrode to perform the plasma discharge, and an insulator that surrounds the ground electrode.

A photocatalyst part or photocatalyst which is activated by visual light to decompose pollutants or reduce an amount of ozone may be disposed on at least one surface of the insulator. The photocatalyst part may include silver phosphate (Ag₃PO₄), titanium dioxide (TiO₂), and an inorganic binder.

The plasma device may be disposed so that air flowing through the inside of the main body flows along one surface of the main body within the main body.

Embodiments disclosed herein further provide a plasma device that may include a substrate body; a first discharge electrode disposed on a top surface of the substrate body to perform plasma discharge; and a second discharge electrode disposed on a bottom surface of the substrate body to generate ions. The substrate body may include a ground electrode that acts with the first or second discharge electrode to perform the plasma discharge, and an insulator that surrounds the ground electrode. Air flowing along the top surface of the substrate body may react with a plasma region defined by the reaction between the first discharge electrode and the ground electrode to generate a plurality of ions.

A photocatalyst part or photocatalyst that may be activated by visual light to decompose pollutants or reduce an amount of ozone may be disposed on at least one surface of the insulator. The photocatalyst part may include silver phosphate (Ag₃PO₄), titanium dioxide (TiO₂), and inorganic binder.

The insulator may be formed of an epoxy resin. The plurality of ions may include hydroxy radicals (OH-).

### BRIEF DESCRIPTION OF THE DRAWINGS

Embodiments will be described in detail with reference to the following drawings in which like reference numerals refer to like elements, and wherein:
Fig. 1 is a front perspective view illustrating a front or first surface of a plasma device according to an embodiment;
Fig. 2 is a rear perspective view illustrating a back or second surface of the plasma device according to an embodiment;
Fig. 3 is a cross-sectional view taken along line III-III' of Fig. 1;
Fig. 4 is a cross-sectional view illustrating a state in which air flows along a surface of the plasma device according to an embodiment;
Fig. 5 is a cross-sectional view of a plasma device according to another embodiment; and
Fig. 6 is a schematic diagram of an air conditioning apparatus according to an embodiment.

### DETAILED DESCRIPTION OF THE EMBODIMENTS

Hereinafter, embodiments disclosed in this specification is described with reference to the accompanying drawings, and the same or corresponding components are given with the same drawing number regardless of reference number, and their duplicated description will be omitted. Furthermore, terms, such as a "module" ad a "unit", are used for convenience of description, and they do not have different meanings or functions in themselves. Moreover, detailed descriptions related to well-known functions or configurations will be ruled out in order not to unnecessarily obscure subject matters of the present disclosure. However, this does not limit the present disclosure within specific embodiments and it should be understood that the present disclosure covers all the modifications, equivalents, and replacements within the idea and technical scope of the present disclosure.

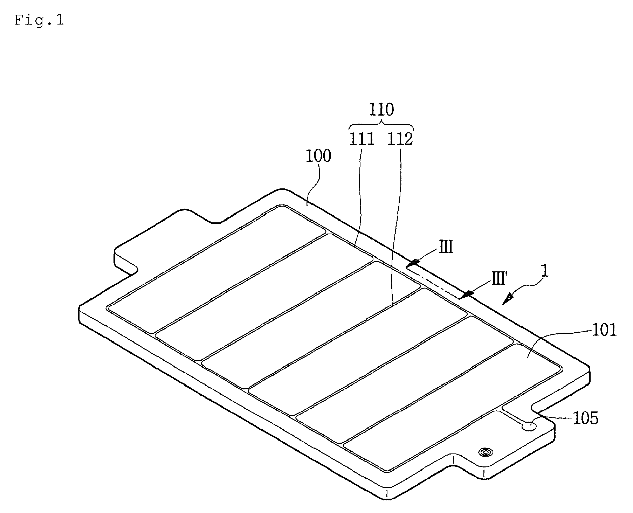

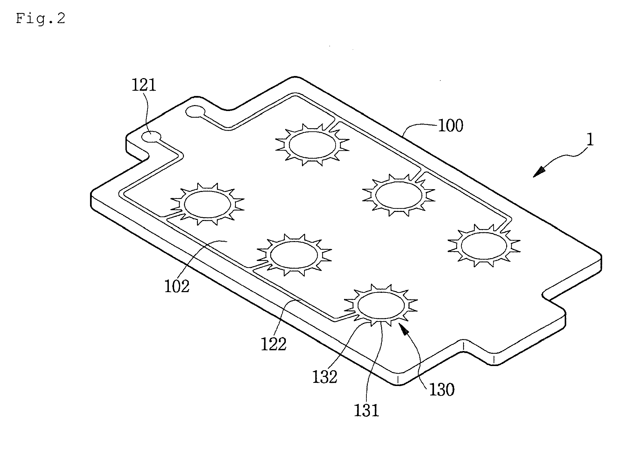

Fig. 1 is a front perspective view illustrating a front or first surface of a plasma device according to an embodiment. Fig. 2 is a rear perspective view illustrating a back or second surface of the plasma device according to an embodiment.

Referring to Figs. 1 and 2, a plasma discharge device 1 according to an embodiment may include a substrate body 100, which may have an approximately square plate shape, a first electrode 110 disposed on one or a first surface of the substrate body 100 to perform plasma discharge, and a second electrode 130 disposed on the other or a second surface of the substrate body 100. The first electrode 110 and the second electrode 130 may be first and second discharge electrodes. The surface on which the first electrode 110 is disposed may be referred to as a "top surface", and a surface on which the second electrode 130 is disposed may be referred to as a "bottom surface".

The first and second electrodes 110 and 130 may be disposed on top and bottom surfaces 101, 102 of one substrate body 100, respectively. The first electrode 110 that performs the plasma discharge may be disposed on a top surface 101 of the substrate body 100. The first electrode 110 may be disposed on the top surface 101 by forming a pattern using a frame 111 that defines an edge of the top surface 101, and a plurality of branches 112 branched from the frame 111. The first electrode 110 may be formed of a metal plate, for example, copper (Cu).

A first electrode pad 105, to which power may be applied, may be disposed on or at a side of the top surface 101. Thus, the first electrode 110 may form a pattern on the top surface 101 through a connection line that extends from the first electrode pad 105 toward a plurality of pattern frames.

The second electrode 130 that generates ions may be disposed on the bottom surface 102 of the substrate body 100. The second electrode 130 may include a second electrode pad 121, to which power may be applied, a pattern frame 131 having at least one pattern shape on the bottom surface 102, and at least one discharge tip 132 disposed on the pattern frame 131.

The pattern frame 131 may have a closed pattern shape, and a plurality of the pattern frame 131 may be provided. For example, six pattern frames 131 may be disposed on the bottom surface 102 in pairs in a longitudinal direction. The pattern shape may include a circular shape, an oval shape, and a polygonal shape, for example. The at least one discharge tip 132 may protrude from an outer circumferential surface of the pattern shape 131.

The second electrode 130 may include a connection line 122 that extends from the second electrode pad 121 toward the plurality of pattern frames 131. The connection line 122 may be branched from the plurality of pattern frames 131.

The second electrode 130 may be formed by printing metal oxide paste, for example. A metal material of the metal oxide paste may be selected from the group consisting of tungsten, iron, copper, platinum, and silver. For example, the metal material may be silver (Ag).

Silver oxide paste may be printed on the bottom surface 102 of the substrate body 100. As the silver oxide paste has a resistance of about 10 Ω to about 20 Ω, the discharge may be easily performed due to the low resistance. Thus, the discharge may be uniformly generated over the electrode. Also, the silver oxide paste may reduce an amount of ozone through the discharge.

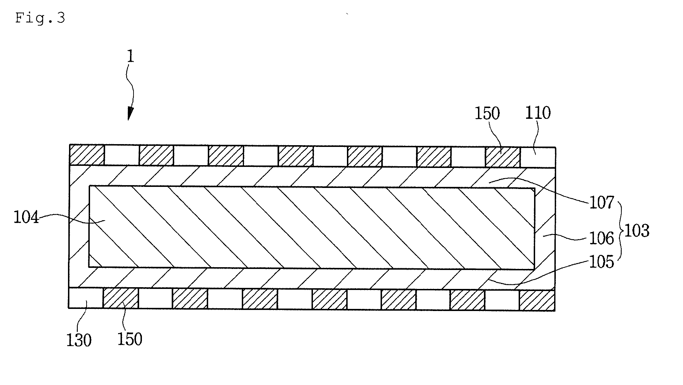

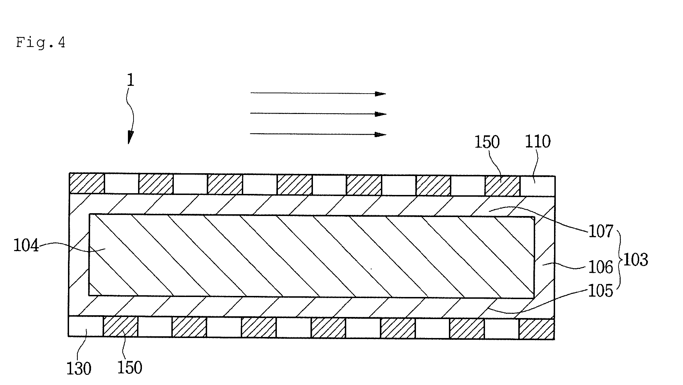

Fig. 3 is a cross-sectional view taken along line III-III' of Fig. 1. Fig. 4 is a cross-sectional view illustrating a state in which air flows along a surface of the plasma device according to an embodiment.

Referring to Fig. 3, the substrate body 100 according to an embodiment may include a third electrode 104, which may be a ground electrode, that interacts with the first electrode 110 or the second electrode 130 to perform the plasma discharge, and an insulator 103 that surrounds the third electrode 104 to prevent the third electrode 104 from being exposed to the outside. The third electrode 104 may be formed of a metal plate, for example, copper (Cu), and the insulator 103 may be formed of an epoxy resin, for example.

A positive potential difference may be created between the first electrode 110 or the second electrode 130 and the third electrode 104. For example, a positive 1 V voltage may be applied to the first electrode 110 or the second electrode 120, where the third electrode is a ground electrode. Alternatively, a positive to negative potential difference may be created between the first electrode 110 or the discharge electrode 130 and the third electrode 104. For example, a positive ½ V voltage may be applied to the first electrode 110 or the discharge electrode 130 and a negative ½ voltage may be applied to the third electrode 104.

The insulator 103 may include a bottom surface 105, on which the third electrode 104 may be seated, a side surface 106 that extends in a upward direction from each of both sides of the bottom surface 105, and a top surface 107 coupled to an upper portion of the side surface 106. An outside of the third electrode 104 may be completely surrounded by the bottom surface 105, the side surface 106, and the top surface 107 of the insulator 103.

A method of manufacturing the third electrode 104 and the insulator 103 will be described hereinafter.

The third electrode 104 may be printed (masked) on an upper portion of the bottom surface 105 of the insulator 103. The bottom surface 105 may be formed of an epoxy resin and may be understood as or referred to as a "base" on which the third electrode 104 may be disposed.

When the third electrode 104 is printed, a bottom surface of the third electrode 104 may be covered by the insulator 103, and side and top surfaces of the third electrode 104 may be exposed to the outside. The side surface 106 and the top surface 107 of the insulator 103 may be applied to the side and top surfaces of the third electrode 104, which may be exposed to the outside, respectively. The applied side surface 106 and top surface 107 may be formed of the same epoxy resin as the bottom surface 105.

A photocatalyst 150 that reacts to visual light or is activated by visual light may be disposed on the top surface 107 and the bottom surface 105 of the substrate body 100. That is, the photocatalyst 150 may be applied on the top surface 107 and the bottom surface 105 of the substrate body 100 except for the surfaces on which the first and second electrodes 110 and 130 are disposed. The photocatalyst 150 may decompose various harmful substances, perform antibacterial and sterilization functions, and reduce an amount of ozone.

The visual light may be understood as or refer to external light existing outside of the plasma device 1. For example, the visual light may include natural light or a lighting source that exists in a predetermined space.

The photocatalyst 150 may include a plurality of composites. The plurality of composites may include silver phosphate (Ag₃PO₄), titanium dioxide (TiO₂), and an inorganic binder. For example, the plurality of composites may include about 20 to about 50 parts by weight of silver phosphate (Ag₃PO₄), about 5 to about 40 parts by weight of titanium dioxide (TiO₂), and about 10 to about 40 parts by weight of the inorganic binder.

Titanium dioxide (TiO₂) may have high activity when UV rays are irradiated and be chemically stable without being eroded by an acid, a base, and an organic solvent. The silver phosphate (Ag₃PO₄) may cause a catalytic activity reaction by optical energy having a visible-ray wavelength range of about 385 nm or more and a mean wavelength of about 500 nm. As the silver phosphate (Ag₃PO₄) is mixed with the titanium dioxide (TiO₂), the photocatalyst 150 may also be effectively activated by the visual light. The silver phosphate (Ag₃PO₄) in itself may have antibacterial (bacteria, mold, for example) performance and a synergy effect, such as decomposition efficiency of organic materials (microorganism, bad small component, for example) through simultaneous activity with titanium dioxide in low energy (the visible-ray wavelength range) by the silver phosphate (Ag₃PO₄).

The inorganic binder may include a polysilicate compound. The polysilicate compound may be composed of colloidal silica (SiO₂) and metal alkoxide, for example.

The inorganic binder may include other additional components. The other components may be selected by a person skilled in the art in consideration of a final composition for a coating. For example, the inorganic binder may include a stabilizer, an acid catalyst, a hardener, and/or a metal additive, for example.

The stabilizer may be selected from the group consisting of acetyl acetone, ethyl acetoacetate, iron acetoacetate, alkanolamine, and a combination thereof. The inorganic binder may contain about 0.1 parts to about 0.5 parts by weight of stabilizer.

The acid catalyst may be selected from the group consisting of a phosphate metal catalyst, a nitrate metal catalyst, a phosphate-chloride composite metal catalyst, and a combination thereof. The inorganic binder may contain about 0.01 parts to about 0.5 parts by weight of acid catalyst.

The hardener may be selected from the group consisting of aliphatic polyamine, crylonitrile-modified amine, polyaminde, amido amine, dicyandiamide, amide resin, isocyanate, melamine, and a combination thereof. The inorganic binder may contain about 0.05 parts to about 1 part by weight of hardener.

An aluminum compound may be used as the metal additive. The aluminum compound may be prepared by mixing aluminum isopropoxide with aluminum chloride. The inorganic binder may contain about 0.05 parts to about 0.5 parts by weight of metal additive.

The photocatalyst 150 may be provided in the form of a solution in which the plurality of composites is mixed with a predetermined solvent. The photocatalyst 150 may be bonded to the bottom surface 105 or the top surface 107 of the insulator 103. Also, the insulator 103 may be bonded to all of the bottom surface 105 and the top surface 107.

For example, the photocatalyst 150 may be bonded to the bottom surface 105 and the top surface 105 by a coating thereof. For example, the coating may include dip coating, spray coating, or screen printing, for example. In the case of the dip coating, a drying temperature may vary according to characteristics of a base material for the coating. For example, the dip coating may be performed at a temperature of about 148°C to about 152°C for about 9 minutes to about 11 minutes.

As described above, the photocatalyst 150 may be prepared in the form of the solution and applied to the bottom surface 105 and the top surface 107. Thus, the photocatalyst 190 may be easily bonded to the bottom surface 105 and the top surface 107 (bonding force securement).

When the photocatalyst 150 containing the above-described composite is disposed on the bottom surface 105 and the top surface 107, water (H₂O) or oxygen (O₂) may change into reactive oxygen species (ROS) due to a catalyst effect of the photocatalyst 150. The reactive oxygen species may include hydroxy radical (OH-), and hydrogen peroxide (H₂O₂), for example.

The reactive oxygen species (ROS) may perform strong sterilization (oxidation) and deodorization functions. In detail, reactive oxygen species (ROS) may decompose gas pollution materials, such as toluene, and ammonia, for example, as well as biological pollution materials, such as bacteria, and molds, for example, which consist of organic materials.

Thus, the photocatalyst 150 may prevent pollutants which are generated by air or moisture from being generated, that is, prevent dust from being accumulated or microorganisms from being propagated.

The second electrode 130 may be formed by printing metal oxide paste, for example.

An operation of the substrate body 100 including the above-described components will be described hereinafter. First, an operation of the second electrode 130 and the third electrode 104 will be described.

When a high potential above the firing voltage is provided between the third electrode 104 and the second electrode 130 including the pattern frame 131, a discharge phenomenon due to high electric fields may occur around the third electrode 104 and the second electrode 130. Free electrons moving around the third electrode 104 and the second electrode 130 may be accelerated by the electric fields to collide with neutral molecules (oxygen, and nitrogen, for example) of the air, thereby ionizing the neutral molecules. Thus, a large amount of ions may be generated. As illustrated in Fig. 4, the air may be air flowing along the top surface 101 of the substrate body 100. The second electrode 130 may be understood as or be referred to as an "ion generation electrode" that generates ions. Based on airflow, the ions may be distributed to the ambient air.

The first electrode 110 that acts with the third electrode 104 to perform the plasma discharge may be disposed on the top surface 101 of the substrate body 100. The first electrode 110 may be formed of a metal plate, for example, copper (Cu).

An operations of the first electrode 110 and the third electrode 104 will be described hereinafter.

When a high potential above the firing voltage is provided between the third electrode 104 and the first electrode 110, dielectric breakdown between the third electrode 104 and the first electrode 110 may occur, causing the discharge phenomenon due to the high electric fields, thereby generating a strong plasma region. Free electrons moving through the plasma region may be accelerated by the electric fields to react with the air. As a result, a large amount of OH radicals may be generated. As illustrated in Fig. 4, the air may be air flowing along the top surface 101 of the substrate body 100. The first electrode 110 may be understood as or be referred to as a "surface discharge induced electrode" that generates radicals.

According to this embodiment, as the plasma electrode is formed by one sheet of electrode having a structure of two sheets of plasma electrodes, manufacturing costs may be reduced, and product processes simplified. Further, pollutants may be removed, or a smell attached to or at a predetermined position may be easily removed using the large amount of generated ions. Furthermore, a smell floating in the air may be removed using the large amount of radicals.

Also, the photocatalyst that reacts with the visual light may be disposed on the plasma device to easily decompose various harmful substances, perform the antibacterial function and sterilization function, and reduce an amount of ozone. Additionally, the insulator formed of an epoxy resin may be disposed to surround the outside of the electrode formed by the metal plate to easily generate the plasma discharge. Also, as one sheet of electrode is provided to allow the air to flow in a direction parallel to a surface of the plasma device, a pressure loss of the air may be reduced.

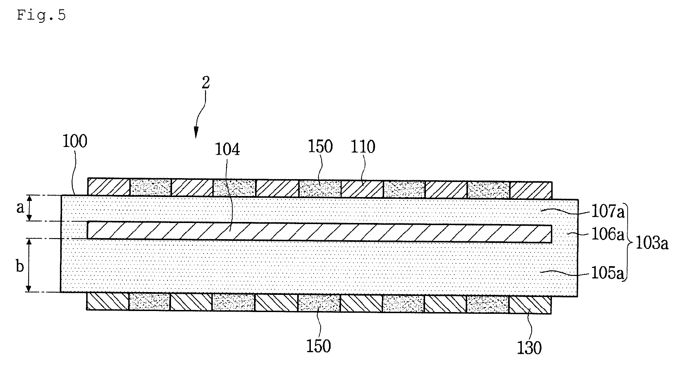

Fig. 5 is a cross-sectional view of a plasma device according to another embodiment. This embodiment may be similar to the previous embodiment except for a thickness of the insulator. Thus, only characterized portions of this embodiment will be described, and descriptions of the same or like portions as those of the previous embodiment have not been repeated.

Referring to Fig. 5, in a plasma device 2 according this embodiment, an insulator 103a has a top surface 107a and a bottom surface 105a, which respectively surround top and bottom surfaces of third electrode 104 and have thicknesses different from each other. If a length from first electrode 110 disposed on a top surface of substrate body 100 to a top surface of the third electrode 104 is defined as reference symbol "a", and a length from second electrode 130 disposed on a bottom surface of the substrate body 100 to a bottom surface of the third electrode 104 is defined as reference symbol "b", the length "a" is less than the length "b". That is, the top surface 107a may have a thickness less than a thickness of the bottom surface 105.

The more the insulator 103a decreases in thickness, the more a high voltage applied to each of the first and second electrodes 110 and 130 decreases in intensity. Also, the first electrode may generate a relatively large amount of ozone when compared to the second electrode 130. Thus, in this embodiment, the top surface 107 may have a thickness less than the thickness of the bottom surface 105a to reduce the amount of ozone generated in the plasma device 2 and the intensity of the applied high voltage.

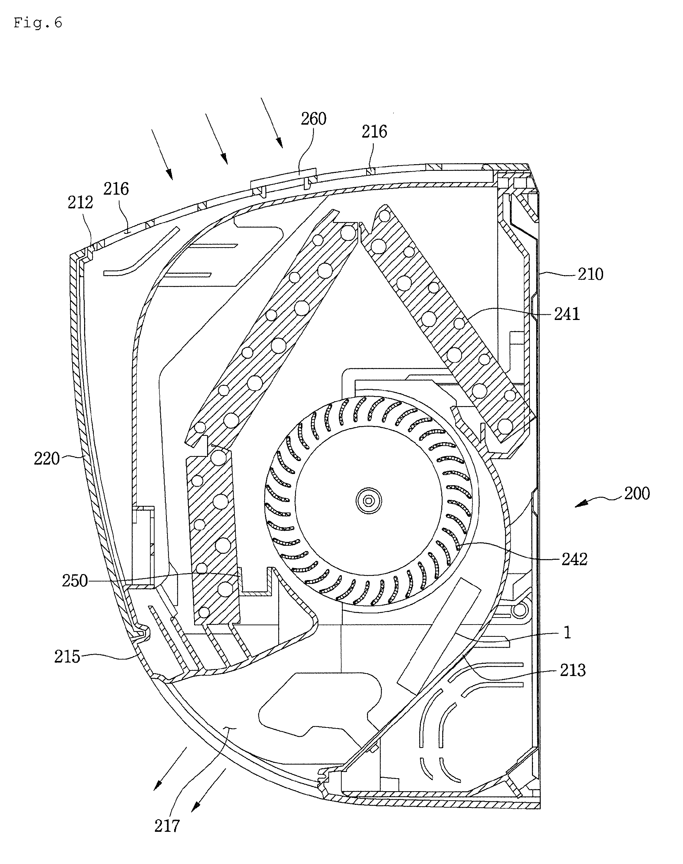

Fig. 6 is a schematic diagram of an air conditioning apparatus according to an embodiment. Referring to Fig. 6, an air conditioning apparatus or air conditioner 200 may include a main body 210, in which a plurality of components may be accommodated.

The main body 210 may include a front frame 212 and a rear frame 213, which may define an exterior of the main body 210. When the front frame 212 and the rear frame 213 are coupled to each other in a front/rear direction, a space in which various components, such as an indoor heat-exchanger 241 and a fan 242 may be installed, may be defined between the front frame 212 and the rear frame 213.

The main body 210 may further include a front panel 220 disposed on a front surface of the front frame 212 to define a front exterior of the main body 210. The front panel 220 may be rotatably coupled to the front frame 212.

The main body 210 may include a suction grill 216a having a suction hole 216, through which indoor air may be suctioned, and a discharge hole 217 through the suctioned in indoor air may be discharged into an indoor space. The suction grill 216a may be disposed in or at an upper portion of the main body 210, substantially, an upper portion of the front frame 212, and the discharge hole 217 may be defined over the front surface and a bottom surface of the main body 210. However, embodiments are not limited to positions of the suction hole 216 and the discharge hole 217.

The main body 210 may include the plasma device 1 to filter the air suctioned in through the suction hole 216, the indoor heat-exchanger 241 in which the indoor air may be heat-exchanged with a refrigerant, the fan 242 to allow the indoor air to forcibly flow, a discharge grill 215 that guides discharge of the indoor air heated-exchanged with the refrigerant, and a charging device or charger 260 that charges dust in the air.

A portion or all of the indoor heat-exchanger 241 may be disposed to be inclined within the main body 210. The indoor heat-exchanger 241 may include a plurality of heat exchangers connected to each other. Alternatively, the indoor heat-exchanger 241 may be provided as a single heat exchanger which is bent several times.

The plasma device 1 may generate a large amount of ions between the fan 242 and the discharge hole 217 to remove pollutants or smell attached to or at a predetermined position. The plasma device 1 may be disposed on one surface of the rear frame 213 within the main body 210.

Alternatively, the plasma device 1 may be disposed between the suction hole 216 and the indoor heat-exchanger 241. In this case, the plasma device 1 may be disposed on the indoor heat-exchanger 241 or on one surface of the front frame 212 having the suction hole 216 within the main body 210. Alternatively, the plasma device 1 may be disposed in a vicinity of each of the suction hole 216 and the discharge hole 217.

When the plasma device 1 is disposed in the vicinity of the suction hole 216, an inside of the air conditioner 200 may be easily sterilized, and floating matter within the air conditioner 200 may be easily removed. When the plasma device 1 is disposed in the vicinity of the discharge hole 217, an amount of ions discharged from the discharge hole 217 may increase. For example, the plasma device 1 may be coupled to the rear frame 213 in a hook manner; however, embodiments are not limited to the coupling structure of the plasma device 1.

The discharge grill 215 may support the indoor heat-exchanger 241. A dust storage portion 250 to collect dust particles removed from the plasma device 1 may be coupled to the discharge grill 215. Alternatively, the discharge grill 215 may define the dust storage portion 250. Alternatively, the dust storage portion 250 may be coupled to an upstream or downstream side of the indoor heat-exchanger 240 with respect to a flow of the air. For example, the dust storage portion 250 may be coupled to the indoor heat-exchanger 240 using a hook.

To collect the dust removed from the plasma device 1 into the dust storage portion 250, the dust storage portion 250 may be disposed under the plasma device 1. As another example, the dust storage portion 250 may be coupled to a lower portion of the plasma device 1, or a portion of the plasma device 1 may be defined as the dust storage portion 250.

The charger 260 may charge dust in the air so that an amount of dust collected by the plasma device 1 increases. The charger 260 may be separably coupled to the suction grill 216a outside of the main body 210.

As the indoor air is suctioned into the main body 210 through the suction hole 216 defined in the suction grill 216a, when the charger 260 is disposed outside of the main body 210, an amount of charged dust in the air may be maximized. Further, as the charger 260 is disposed outside of the main body 210, utilization of space within the main body 210 may be improved. Also, the charger 260 may change according to an installed position of the main body 210.

A plasma device according to embodiments disclosed herein may have at least the following advantages.

First, as the plasma device is formed by one sheet of electrode having a structure of two sheets of plasma electrodes, manufacturing costs may be reduced, and product processes may be simplified. Second, pollutants may be removed, or a smell attached to or at a predetermined position may be easily removed using the large amount of generated ions. Also, a smell floating in the air may be removed using the large amount of radicals.

Third, the photocatalyst that reacts with visual light may be disposed on the plasma device to easily decompose various harmful substances, perform the antibacterial function and sterilization function, and reduce an amount of ozone. Fourth, the insulator formed of an epoxy resin may be disposed to surround the outside of the electrode formed by the metal plate to easily generate the plasma discharge. Fifth, as one sheet of electrode is provided to allow the air to flow in a direction parallel to one surface of the plasma device, pressure loss of the air may be reduced.

Although embodiments have been described with reference to a number of illustrative embodiments thereof, it should be understood that numerous other modifications and embodiments can be devised by those skilled in the art that will fall within the spirit and scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A plasma device, comprising:
a substrate body;
a first electrode provided on the substrate body; and
a second electrode provided on the substrate body that acts with the first electrode to perform plasma discharge in an air flow that extends along a surface of the plasma device substantially parallel to a central longitudinal axis of the plasma device.

2. The plasma device according to claim 1, wherein the second electrode is a ground electrode.

3. The plasma device according to claim 2, further comprising an insulator that surrounds the ground electrode.

4. The plasma device according to claim 3, wherein the insulator comprises:
a base on which the ground electrode is seated;
a side surface that extends from each of both sides of the base to surround a side surface of the ground electrode; and
a top surface that extends from the side surface to surround a top surface of the ground electrode.

5. The plasma device according to claim 3, wherein the insulator is formed of an epoxy resin.

6. The plasma device according to claim 3, wherein a photocatalyst which is activated by visual light is disposed on at least one surface of the insulator.

7. The plasma device according to claim 6, wherein the photocatalyst at least one of decomposes pollutants or reduces ozone.

8. The plasma device according to claim 6, wherein the photocatalyst comprises silver phosphate (Ag₃PO₄), titanium dioxide (TiO₂), and an inorganic binder.

9. The plasma device according to claim 1, further comprising a third electrode that acts with the second electrode to generate a plurality of ions.

10. The plasma device according to claim 9, wherein the first electrode is provided on a first surface of the substrate body, and wherein the third electrode is provided on a second surface of the substrate body.

11. The plasma device according to claim 10, wherein the first surface is opposite to the second surface

12. The plasma device according to claim 11, wherein the third electrode includes:
a discharge electrode pad to which power is applied;
at least one pattern frame disposed on a surface of the substrate body; and
at least one discharge tip disposed on the at least one pattern frame.

13. The plasma device according to claim 11, wherein the first electrode comprises a frame that defines an edge of the first surface of the substrate body, and a plurality of branches branched from the frame to form a pattern.

14. The plasma device according to claim 11, wherein a first electrode pad, to which power is applied, is provided on the first surface of the substrate body, and wherein the first electrode is connected to a connection line that extends from the first electrode pad.

15. The plasma device according to claim 11, wherein a second electrode pad, to which power is applied, is provided on the second surface of the substrate body, and wherein the third electrode is connected to a connection line that extends from the second electrode pad.
